(19) Europäisches Patentamt
European Patent Office
Office européen des brevets

(11) EP 0 569 802 B1

(12) EUROPÄISCHE PATENTSCHRIFT

(45) Veröffentlichungstag und Bekanntmachung des Hinweises auf die Patenterteilung:
**15.07.1998 Patentblatt 1998/29**

(21) Anmeldenummer: **93107103.9**

(22) Anmeldetag: **01.05.1993**

(51) Int Cl.[6]: **C07D 207/12**, C07D 217/06, C07D 217/14, C07D 295/12, C07D 311/84, C07D 401/06, C07D 405/06, A61K 31/40, A61K 31/47, A61K 31/35

# (54) Arylacetamide

Arylacetamides

Arylacétamides

(84) Benannte Vertragsstaaten:
**AT BE CH DE DK ES FR GB GR IE IT LI LU NL PT SE**

(30) Priorität: **09.05.1992 DE 4215213**

(43) Veröffentlichungstag der Anmeldung:
**18.11.1993 Patentblatt 1993/46**

(73) Patentinhaber: **MERCK PATENT GmbH**
**64271 Darmstadt (DE)**

(72) Erfinder:
- **Gottschlich, Rudolf, Dr.**
  **W-6109 Reinheim (DE)**
- **Ackermann, Karl-August**
  **W-6105 Ober-Ramstadt (DE)**
- **Prücher, Helmut**
  **W-6148 Heppenheim (DE)**
- **Seyfried, Christoph, Dr.**
  **W-6104 Seeheim (DE)**
- **Greiner, Hartmut, Dr.**
  **W-6100 Darmstadt (DE)**
- **Bartoszyk, Gerd**
  **W-6100 Darmstadt (DE)**
- **Mauler, Frank, Dr.**
  **W-6104 Seeheim (DE)**
- **Stohrer, Manfred, Dr.**
  **W-6500 Mainz 1 (DE)**
- **Barber Andrew, Dr.**
  **W-6108 Weiterstadt (DE)**

(56) Entgegenhaltungen:

| | |
|---|---|
| **EP-A- 0 232 989** | **EP-A- 0 330 467** |
| **EP-A- 0 374 756** | **EP-A- 0 483 580** |
| **WO-A-91/08205** | **WO-A-91/08206** |
| **FR-A- 2 421 891** | **US-A- 4 192 883** |

- **Rec. Trav. Chim. Pays-Bas, 1993, 112 (2), 169-73**
- **Exp. Opin. Invest. Drugs, 1994, 3 (4), 369-76**
- **J. Med. Chem., 1982, 25, 1125-6**
- **Br. J. Pharmacol., 1994, 113, 1317-27**

EP 0 569 802 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue Arylacetamide der Formel I

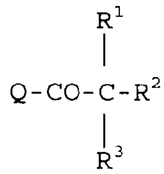

I

worin

Q $R^4$-$CH(CH_2Z)$ -NA-,

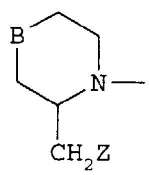

,

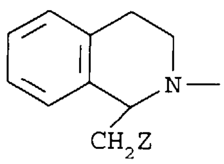

oder

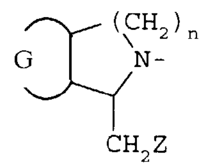

,

$R^1$ Ar,

$R^2$ Ar,

$R^1$ und $R^2$ zusammen auch

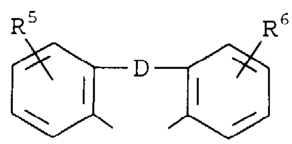

,

$R^3$ H, OH, OA oder A,

$R^4$ A oder Phenyl, welches gegebenenfalls ein- oder zweifach durch F, Cl, Br, I, OH, OA, $CF_3$, $NO_2$, $NH_2$, NHA, NHCOA, $NHSO_2A$ oder $NA_2$ substituiert sein kann,

$R^5$ und $R^6$ jeweils unabhängig voneinander H, F, Cl, Br, I, OH, OA, $CF_3$, $NH_2$, NHA, $NA_2$, NHCOA, $NHCONH_2$, $NO_2$ oder Methylendioxy,

A Alkyl mit 1-7 C-Atomen,

B $CH_2$, O, NH, NA, N-COA, N-COOA oder eine Bindung,

G ein ankondensiertes Ringsystem mit 3-5 C-Atomen, wobei gegebenenfalls ein C-Atom durch S, N oder O ersetzt sein kann und welches gegebenenfalls ein- oder zweifach durch F, Cl, Br, I, OH, OA, $NH_2$, NHA, $NA_2$, NH-COA, NA-COA oder NH-$CONH_2$ substituiert sein kann,

D $CH_2$, O, S, NH, NA, -$CH_2$-$CH_2$-, -CH=CH-, -$CH_2O$-, -$CH_2NH$-, -$CH_2NA$- oder eine Bindung,

Z 1-Pyrrolidinyl, welches gegebenenfalls einfach durch OH, OA, O-$COCH_3$ oder $CH_2OH$ substituiert sein kann,

Ar unsubstituiertes oder einfach durch $NH_2$, OH, $NHCOCH_3$, F, Cl oder $CF_3$, substituiertes Phenyl,

-alk einen Alkylenrest mit 1-7 Atomen

und

n 1 oder 2

bedeuten,
sowie deren Salze.

Ähnliche Verbindungen sind in DE-A1-39 35 371 beschrieben.

Pyrrolidine mit ähnlichen pharmakologischen Eigenschaften aber anderen Substitutionsmustern sind z.B. in der EP 0 483 580 beschrieben.

N-Acyl substituierte azacyclische Verbindungen, die als Kappa-Rezeptor-Agonisten wirken, kennt man z.B. aus WO 91/08206 und WO 91/08205, andere heterocyclische Substanzen mit dergleichen Wirkung aus EP 0 330 467.

Ähnliche Isochinolinderivate mit anderem Substitutionsmuster sind in der EP 0 232 989 beschrieben.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze wertvolle pharmakologische Eigenschaften besitzen. Sie zeigen eine analgetische Wirkung und antagonisieren insbesondere die entzündungsbedingte Hyperalgesie. So wirken die Verbindungen im "Writhing Test" an Mäusen oder Ratten (Methode vgl. Siegmund et al., Proc. Soc. Exo. Biol. 95, (1957), 729-731). Die analgetische Wirkung läßt sich ferner im "Tail-Flick-Test" an Mäusen oder Ratten nachweisen (Methodik vgl. d'Amour und Smith, J. Pharmacol. Exp. Ther. 72, (1941), 74-79), ferner im "Hot plate test" (vgl. Schmauss und Yaksh, J. Pharmacol. Exp. Ther. 228, (1984), 1-12 und die dort zitierte Literatur). Besonders starke Wirkungen sind an Ratten im Modell der Carrageenin-induzierten Hyperalgesie (vgl. Bartoszyk und Wild, Neuroscience Letters 101 (1989) 95) zu beobachten. Dabei zeigen die Verbindungen keine oder nur geringe Neigung zu physischer Abhängigkeit. Außerdem treten antiinflammatorische, antiasthmatische, diuretische, antikonvulsive, neuroprotektive und/oder antitussive Wirkungen auf, die ebenfalls nach hierfür geläufigen Methoden nachgewiesen werden können. Die Verbindungen zeigen eine hohe Affinität in bezug auf das Bindungsverhalten an kappa-Rezeptoren. Sie eignen sich ferner zum Schutz vor und zur Behandlung von Hirnödemen und Unterversorgungszuständen des Zentralnervensystems, vor allem Hypoxie.

Die Verbindungen können daher als Arzneimittelwirkstoffe in der Human- und Veterinärmedizin verwendet werden. Ferner eignen sie sich als Zwischenprodukte zur Herstellung anderer Verbindungen mit wertvollen Eigenschaften.

Gegenstand der Erfindung sind Verbindungen der Formel I sowie ihre Salze.

Die Gruppe A steht für Alkyl mit 1, 2, 3, 4, 5, 6 oder 7 C-Atomen, insbesondere für Methyl oder Ethyl aber auch für Propyl, Isopropyl, Butyl, Isobutyl, sek.-Butyl, tert.-Butyl. Die Gruppe OA ist dementsprechend vorzugsweise Methoxy oder Ethoxy, ferner Propoxy, Isopropoxy, Butoxy, Isobutoxy, sek.-Butoxy oder tert.-Butoxy, die Gruppe -NA- vorzugsweise N-Methyl-, die Gruppe -NHA Methyl-NH und die Gruppe $-NA_2$ N,N-Dimethylamino.

Dementsprechend haben die nachstehenden Gruppen die im folgenden genannten bevorzugten Bedeutungen:

-NH-CO-A: Acetamido, Propionamido;
-NA-CO-A: N-Methylacetamido, N-Methylpropionamido.

Ar ist bevorzugt unsubstituiertes Phenyl, ferner bevorzugt o-, m- oder p-Aminophenyl, weiterhin bevorzugt o-, m- oder p-Hydroxyphenyl, o-, m- oder p-Acetamidophenyl, o-, m- oder p-Fluorphenyl, o-, m- oder p-Chlorphenyl, o-, m- oder p-Trifluormethyl-phenyl. Unter den substituierten Phenylresten sind die in p-Stellung, aber auch die in m-Stellung bevorzugt.

Die Gruppe -alk bedeutet vorzugsweise $-CH_2-$ oder $-CH_2-CH_2-$.

$R^1$ und $R^2$ sind jeweils unabhängig voneinander besonders bevorzugt Phenyl, ferner auch p-Fluorphenyl oder p-Chlorphenyl.

Ebenso können $R^1$ und $R^2$ vorzugsweise auch über ihre ortho-Positionen durch eine direkte Bindung oder über eine O- oder eine Methylenbrücke miteinander verbunden sein.

$R^3$ bedeutet besonders bevorzugt H oder OH, ferner auch OA oder Methyl.

$R^4$ bedeutet vorzugsweise Phenyl, p-Hydroxyphenyl, p-Methoxyphenyl, ferner p-F-, p-Cl- oder p-Trifluormethylphenyl, aber auch Alkyl, wie z.B. Methyl, Ethyl, Propyl, Isopropyl, Butyl, Isobutyl, sek.-Butyl, tert.-Butyl oder Pentyl.

$R^5$ und $R^6$ bedeuten vorzugsweise jeweils unabhängig voneinander Wasserstoff, F oder Cl, ferner OH oder Methoxy.

Der Rest Q besitzt vorzugsweise die folgenden Bedeutungen:

N-Methyl-N-(1-phenyl-2-pyrrolidino-ethyl-amino;
N-Methyl-N-[1-phenyl-2-(3-hydroxy-pyrrolidino)-ethyl]-amino;
N-Methyl-N-(1-p-hydroxyphenyl-2-pyrrolidino-ethyl-amino;

N-Methyl-N-[1-p-hydroxyphenyl-2-(3-hydroxy-pyrrolidino)-ethyl]-amino
N-Methyl-N-[1-(p-methoxyphenyl)-2-pyrrolidino-ethyl]-amino;
N-Methyl-N-[1-(p-methoxyphenyl)-2-(3-hydroxy-pyrrolidino)-ethyl]-amino;
N-Methyl-N-(2-pyrrolidino-3-methyl-butyl)-amino;
N-Methyl-N-[2-(3-hydroxy-pyrrolidino)-3-methyl-butyl]-amino;
N-Methyl-N-(2-pyrrolidino-4-methyl-pentyl)-amino;
N-Methyl-N-[2-(3-hydroxy-pyrrolidino)-4-methyl-pentyl]-amino;
2-(Pyrrolidino-methyl)-piperidino;
2-(3-Hydroxy-pyrrolidino-methyl)-piperidino;
2-(Pyrrolidino-methyl)-4-ethoxycarbonyl-piperazino;
2-(3-Hydroxy-pyrrolidino-methyl)-4-ethoxycarbonyl-piperazino;
2-(Pyrrolidino-methyl)-1,2,3,4-tetrahydroisochinolin-1-yl;
2-(3-Hydroxy-pyrrolidino-methyl)-1,2,3,4-tetrahydroisochinolin-1-yl;
2-(Pyrrolidino-methyl)-pyrrolidino oders 2-(3-Hydroxy-pyrrolidino-methyl)-pyrrolidino.

Z bedeutet Pyrrolidino, welches vorzugsweise unsubstituiert oder in 3-Position durch OH substituiert ist; ferner aber auch durch OA, $-O-COCH_3$ oder $-CH_2OH$ substituiert sein kann.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ih ausgedrückt werden, die der Formel I entsprechen und worin die nicht näher bezeichneten Reste die bei der Formel I angegebene Bedeutung haben, worin jedoch

in Ia $R^1$ und $R^2$ jeweils Phenyl bedeuten;

in Ib $R^1$ und $R^2$ jeweils p-Fluor- oder p-Chlorphenyl bedeuten;

in Ic $R^1$ und $R^2$ jeweils Phenyl und $R^3$ H bedeuten;

in Id $R^1$ und $R^2$ jeweils Phenyl und $R^3$ Methyl bedeuten;

in Ie $R^1$ und $R^2$ jeweils p-Fluor- oder p-Chlorphenyl und $R^3$ H bedeuten;

in If $R^1$ und $R^2$ zusammen
bedeuten;

in Ig $R^1$ und $R^2$ zusammen
bedeuten;

in Ih $R^1$ und $R^2$ zusammen die in If oder Ig angegebene Bedeutung haben und $R^3$ H bedeutet

Weiterhin sind bevorzugt Verbindungen der Formeln I', sowie Ia' bis Ih', die den Formeln I bzw. Ia bis Ih entsprechen, worin jedoch jeweils zusätzlich Q

(a) N-Methyl-N-(1-phenyl-2-pyrrolidino-ethyl)-amino;
(b) N-Methyl-N-[1-phenyl-2-(3-hydroxy-pyrrolidino)-ethyl]-amino;
(c) N-Methyl-N-[1-(p-hydroxyphenyl)-2-pyrrolidino-ethyl)-amino;
(d) N-Methyl-N-[1-(p-hydroxyphenyl)-2-(3-hydroxy-pyrrolidino)-ethyl]-amino;
(e) N-Methyl-N-[1-(p-methoxyphenyl)-2-pyrrolidino-ethyl]-amino;
(f) N-Methyl-N-[1-(p-methoxyphenyl)-2-(3-hydroxy-pyrrolidino)-ethyl]-amino;
(g) N-Methyl-N-(2-pyrrolidino-3-methyl-butyl)-amino;
(h) N-Methyl-N-[2-(3-hydroxy-pyrrolidino)-3-methyl-butyl]-amino;
(i) N-Methyl-N-(2-pyrrolidino-4-methyl-pentyl)-amino;
(k) N-Methyl-N-[2-(3-hydroxy-pyrrolidino)-4-methyl-pentyl]-amino;
(l) 2-(Pyrrolidino-methyl)-piperidino;
(m) 2-(3-Hydroxy-pyrrolidino-methyl)-piperidino;
(n) 2-(Pyrrolidino-methyl)-4-ethoxycarbonyl-piperazino;
(o) 2-(3-Hydroxy-pyrrolidino-methyl)-4-ethoxycarbonyl-piperazino;

(p) 2-(Pyrrolidino-methyl)-1,2,3,4-tetrahydroisochinolin-1-yl;
(q) 2-(3-Hydroxy-pyrrolidino-methyl)-1,2,3,4-tetrahydroisochinolin-1-yl;
(r) 2-(Pyrrolidino-methyl)-pyrrolidino oder
(s) 2-(3-Hydroxy-pyrrolidino-methyl)-pyrrolidino bedeutet.

Gegenstand der Erfindung ist ferner ein Verfahren zur Herstellung von Arylacetamiden der Formel I gemäß Anspruch 1 sowie von deren Salzen, dadurch gekennzeichnet, daß man eine Verbindung der Formel II

Q-H II

worin Q die bei der Formel I angegebene Bedeutung hat mit einer Verbindung der Formel III

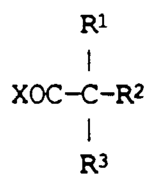

III

worin

X Cl, Br, OH, OA, $NH_2$, $-N_3$, Acyloxy, Ar-alkoxy mit 7-11 C-Atomen oder Aroyloxy mit 6-10 C-Atomen bedeutet

und
$R^1$, $R^2$ und $R^3$ die bei Formel I angegebenen Bedeutungen haben, umsetzt,

oder daß man in einer Verbindung der Formel I einen Rest Q, $R^1$, $R^2$ und/oder $R^3$ in einen anderen Rest Q, $R^1$, $R^2$ und/oder $R^3$ umwandelt,

oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,

und/oder daß man eine basische Verbindung der Formel I durch Behandeln mit einer Säure in eines ihrer Salze umwandelt.

Die Verbindungen der Formel I werden in der Regel nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe sind in der Regel bekannt, oder sie können in Analogie zu bekannten Stoffen nach an sich bekannten Verfahren hergestellt werden. Sie können gewünschtenfalls auch in situ gebildet werden, derart, daß man aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die einzelnen Verfahrensvarianten werden im folgenden näher erläutert.

Die Verbindungen der Formel I sind bevorzugt herstellbar durch Reaktion der Verbindungen der Formel II mit Carbonsäuren der Formel III oder ihren funktionellen Derivaten. Als funktionelle Derivate der Verbindungen der Formel III eignen sich insbesondere die entsprechenden Ester, vor allem die Methyl- oder Ethylester, die Halogenide, Anhydride oder Azide; die Chloride sind bevorzugt.

Verbindungen der Formel II sind beispielsweise erhältlich durch Umsetzung von 1-(Chlormethyl)-1,2,3,4-tetrahydroisochinolin mit Pyrrolidin oder 3-Hydroxypyrrolidino-ethan, von 1-Amino-1-phenyl-2-pyrrolidino-ethan mit Methyliodid, von 1-N-Methylamino-1-phenyl-2-halogen-ethan (Halogen entspricht vorzugsweise Cl od. Br) mit Pyrrolidin oder 3-Hydroxypyrrolidin oder von 1-Halogen-2-N-methylamino-4-methyl-pentan mit Pyrrolidin oder dessen 3-Hydroxyderivat.

Ferner kann man Verbindungen der Formel II auch durch Umsetzung von 2-Halogenmethylderivaten des Piperazins oder Piperidins mit Pyrrolidin oder 3-Hydroxypyrrolidin erhalten.

Typische Verbindungen der Formel III sind z.B. Diphenylacetylchlorid, -bromid und -azid, Diphenylessigsäure-methyl- und -ethylester, (Diphenylessigsäure)-anhydrid, Diphenylacetonitril sowie die entsprechenden Derivate der Di-(p-Cl-phenyl)-u. Di-(p-F-phenyl)-essigsäure sowie die entsprechenden Derivate der Hydroxy-diphenylessigsäure bzw. der 2,2-Diphenylpropionsäure.

Die Umsetzung von II mit III bzw. III-Derivaten gelingt zweckmäßig in Anwesenheit oder Abwesenheit eines inerten organischen Lösungsmittels, z.B. eines halogenierten Kohlenwasserstoffs wie Dichlormethan, Chloroform oder Trichlorethen, eines Alkohols wie Methanol, Ethanol oder Butanol, eines Ethers wie Tetrahydrofuran (THF) oder Dioxan, eines Amids wie Dimethylformamid (DMF), eines Sulfoxids wie Dimethylsulfoxid (DMSO) und/oder in Gegenwart oder Abwesenheit eines Kondensationsmittels, z.B. einer Base, bei Temperaturen zwischen -20 und 200°, vorzugsweise 0 und 100°. Als Basen eignen sich z.B. Alkalimetallhydroxide wie NaOH oder KOH, Alkalimetallcarbonate wie $Na_2CO_3$ oder $K_2CO_3$, tertiäre Amine wie Triethylamin oder Pyridin. Als Lösungsmittel ist Dichlomethan, als Base Triethylamin besonders bevorzugt.

Ferner kann man in einer Verbindung der Formel I einen oder mehrere der Reste Q, $R^1$, $R^2$ und/oder $R^3$ in einen oder mehrere andere Reste Q, $R^1$, $R^2$ und/oder $R^3$ umwandeln.

So kann man Ethergruppen (z.B. OA-Gruppen) unter Bildung von OH-Gruppen spalten, z.B. durch Behandeln mit Dimethylsulfid-Bortribromid-Komplex, z.B. in Toluol, THF oder DMSO, oder durch Verschmelzen mit Pyridin- oder Anilinhydrohalogeniden, vorzugsweise Pyridinhydrochlorid, bei etwa 150-250°, oder durch Behandeln mit Diisobutylaluminiumhydrid in Toluol bei etwa 0-110°.

Weiterhin kann man OH-Gruppen verethern, z.B. indem man zunächst die entsprechenden Alkalimetall- (z.B. Na- oder K-)alkoholate, -phenolate oder Salze herstellt und diese mit entsprechenden Halogenverbindungen umsetzt, z. B. mit Alkylhalogeniden wie Methylchlorid, -bromid oder -iodid, Chlor- oder Bromacetamid, zweckmäßig in Gegenwart eines der oben angegebenen Lösungsmittel bei Temperaturen zwischen 0 und 100°.

Nitrogruppen können zu Aminogruppen reduziert werden, zweckmäßig durch katalytische Hydrierung unter den oben angegebenen Bedingungen, z.B. mit Raney-Ni im Methanol oder Ethanol bei 15-40° und Normaldruck.

Aminogruppen können acyliert werden, z.B. mit Säurechloriden wie Acetyl-, Methansulfonyl-, Oxalsäure- oder Bernsteinsäurehalbester-chlorid, zweckmäßig in inerten Lösungsmitteln wie Dichlormethan bei 15-40°.

Ferner können Aminogruppen nach an sich bekannten Methoden alkyliert werden.

Eine Base der Formel I kann weiterhin mit einer Säure in das zugehörige Säureadditionssalz überführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optischaktiven Camphersulfonsäuren wie β-Camphersulfonsäure.

Vorteilhaft ist auch eine Enantiomerentrennung mit Hilfe einer mit einem optisch aktiven Trennmittel (z.B. Dinitrobenzoyl-phenyl-glycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch-aktive Verbindungen der Formel I nach den oben beschriebenen Methoden zu erhalten, indem man Ausgangsstoffe (z.B. solche der Formel II) verwendet, die bereits optisch-aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/ oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, insbesondere von Schmerzzuständen aber auch zur Minderung der Folgeschäden nach einer Ischämie verwendet werden.

Dabei werden die erfindungsgemäßen Substanzen in der Regel in Analogie zu bekannten Analgetika verabreicht, vorzugsweise in Dosierungen zwischen etwa 1 und 500 mg, insbesondere zwischen 5 und 100 mg pro Dosierungseinheit. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. Die Verbindungen der Formel I neigen bei Erhitzen zur Zersetzung, so daß keine eindeutigen Schmelzpunkte ermittelt werden können und dafür ersatzweise die entsprechenden $R_f$-Werte (Dünnschichtchromatographie) angegeben werden. In den nachfolgenden Beispielen bedeutet "übliche Aufarbeitung": Man gibt, falls erforderlich, Wasser oder verdünnte Natronlauge hinzu, extrahiert mit Dichlormethan, trennt ab, trocknet die organische Phase mit Natriumsulfat, filtriert, dampft ein und reinigt durch Chromatographie an Kieselgel und/oder durch Kristallisation.

HCl' = Hydrochlorid. Rf = Rf-Wert auf Dünnschicht-Kieselgel 60 $F_{254}$ (E. Merck, Art.-Nr. 5715), $CH_2Cl_2/CH_3OH$ 9:1
$[\alpha] = [\alpha]_D^{20}$ , c = 1 in Methanol.

Beispiel 1

Eine Lösung von 2,3 g Diphenylessigsäurechlorid in 100 ml THF wird bei Raumtemperatur mit 2,2 g (1S)-1-Methylamino-1-phenyl-2-((3S)-3-hydroxy-pyrrolidino)-ethan [erhältlich aus (1S)-1-Amino-1-phenyl-2-chlorethan durch Umsetzung mit (3S)-3-Hydroxypyrrolidin und anschließender Methylierung mit Methyliodid], gelöst in 20 ml THF, tropfenweise versetzt und 10 Minuten gerührt. Nach üblicher Aufarbeitung erhält man N-Methyl-N-[(1S)-1-phenyl-2-((3S)-3-hydroxy-pyrrolidino)-ethyl]-2,2-diphenylacetamid, Rf: 0,60.

Analog erhält man durch Umsetzung von Diphenylessigsäurechlorid

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-diphenylacetamid, Rf: 0,61;

mit (1S)-1-Methyl-amino-1-phenyl-2-pyrrolidino-ethan:
N-Methyl-N-[(1S)-1-phenyl-2-pyrrolidino-ethyl]-2,2-diphenylacetamid, Rf: 0,71;

mit (1S)-1-Methyl-amino-1-(o-methoxy-phenyl)-2-((3S)-3-hydroxy-pyrrolidino)-ethan:
N-Methyl-N-[(1S)-1-(o-methoxy-phenyl)-2-((3S)-3-hydroxy-pyrrolidino)-ethyl]-2,2-diphenylacetamid;

mit 1-(Pyrrolidino-methyl)-1,2,3,4-tetrahydroisochinolin:
2-Diphenylacetyl-1-(pyrrolidino-methyl)-1,2,3,4-tetrahydroisochinolin, Rf: 0,50;

mit 2-(Pyrrolidino-methyl)-pyrrolidin:
(2S)-1-Diphenylacetyl-2-(pyrrolidino-methyl)-pyrrolidin, Rf: 0,20;

mit N-Methyl-N-(2-pyrrolidino-4-methyl-pentyl)-amin:
N-Methyl-N-(2-pyrrolidino-4-methyl-pentyl)-2,2-diphenylacetamid, Rf: 0,61;

mit N-Methyl-N-[2-((3S)-hydroxy-pyrrolidino)-4-methyl-pentyl]-amin:
N-Methyl-N-[2-((3S)-hydroxy-pyrrolidino)-4-methyl-pentyl]-2,2-diphenyl-acetamid, Rf: 0,64;

mit N-Methyl-N-(2-pyrrolidino-3-methyl-butyl)-amin:
N-Methyl-N-[2-pyrrolidino-3-methyl-butyl)-2,2-diphenylacetamid;

mit N-Methyl-N-[2-((3S)-hydroxy-pyrrolidino)-3-methyl-butyl]-amin:
N-Methyl-N-[2-((3S)-hydroxy-pyrrolidino)-3-methyl-butyl]-2,2-diphenyl-acetamid;

mit (1S)-1-Methyl-amino-1-(p-hydroxy-phenyl)-2-((3S)-3-hydroxy-pyrrolidino)-ethan:
N-Methyl-N-[(1S)-1-(p-hydroxy-phenyl)-2-((3S)-3-hydroxy-pyrrolidino)-ethyl-2,2-diphenylacetamid;

mit (1S)-1-Methyl-amino-1-(p-chlor-phenyl)-2-pyrrolidino-ethan:
N-Methyl-N-[(1S)-1-(p-chlor-phenyl)-2-pyrrolidino-ethyl]-2,2-diphenylacetamid;

mit 1-((3S)-3-Hydroxy-pyrrolidino-methyl)-1,2,3,4-tetrahydroisochinolin:
2-Diphenylacetyl-1-((3S)-3-hydroxy-pyrrolidino-methyl)-1,2,3,4-tetrahydro-isochinolin;

mit (1S)-1-Methyl-amino-1(2,4-di-methoxy-phenyl)-2-((3S)-3-hydroxy-pyrrolidino)-ethan:
N-Methyl-N-[1S)-1-(2,4-di-methoxy-phenyl)-2-((3S)-3-hydroxypyrrolidino)-ethyl]-2,2-diphenylacetamid;

mit (1S)-1-Methyl-amino-1-(p-trifluormethyl-phenyl)-2-((3S)-3-hydroxy-pyrrolidino) -ethan:
N-Methyl-N-[(1S)-1-(p-trifluormethyl-phenyl)-2-(3S)-3-hydroxy-pyrrolidino)-ethyl]-2,2-diphenylacetamid;

mit (1S)-1-Methyl-amino-1-(p-methyl-phenyl)-2-((3S)-3-hydroxy-pyrrolidino)-ethan:
N-Methyl-N-[(1S)-1-(p-methyl-phenyl)-2-((3S)-3-hydroxy-pyrrolidino)-ethyl]-2,2-diphenylacetmid;

mit (1S)-1-Methyl-amino-1-(p-amino-phenyl)-2-((3S)-3-hydroxy-pyrrolidino)-ethan:
N-Methyl-N-[(1S)-1-(p-amino-phenyl)-2-((3S)-3-hydroxy-pyrrolidino)-ethyl]-2,2-diphenylacetamid;

mit (1S)-1-Methyl-amino-1-(p-fluor-phenyl)-2-((3S)-3-hydroxy-pyrrolidino)-ethan:
N-Methyl-N-[(1S)-1-(p-fluor-phenyl)-2-((3S)-3-hydroxy-pyrrolidino)-ethyl]-2,2-diphenylacetamid;

mit (1S)-1-Methyl-amino-1-(2,4-di-fluor-phenyl)-2-((3S)-3-hydroxy-pyrrolidino)-ethan:
N-Methyl-N-[(1S)-1-(2,4-difluor-phenyl)-2-((3S)-3-hydroxy-pyrrolidino)-ethyl]-2,2-diphenylacetamid.

Beispiel 2

Analog Beispiel 1 erhält man durch Umsetzung von Bis-(p-Fluor-phenyl)-essigsäure

mit (1S)-1-Methyl-amino-1-phenyl-2-((3S)-3-hydroxy-pyrrolidino)-ethan:
N-Methyl-N-[(1S)-1-phenyl-2-((3S)-3-hydroxy-pyrrolidino)-ethyl]-2,2-bis-(p-fluor-phenyl)-acetamid;

mit (1S)-1-Methyl-amino-1-(p-methoxy-phenyl)-2-(3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-bis-(p-fluor-phenyl)-acetamid;

mit (1S)-1-Methyl-amino-1-phenyl-2-pyrrolidino-ethan:
N-Methyl-N-[(1S)-1-phenyl-2-pyrrolidino-ethyl]-2,2-bis-(p-fluor-phenyl)-acetamid;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-bis-(p-fluor-phenyl)-acetamid;

mit 1-(Pyrrolidino-methyl)-1,2,3,4-tetrahydroisochinolin:
2-Bis-(p-fluor-phenyl)-acetyl-1-(pyrrolidino-methyl)-1,2,3,4-tetrahydro-isochinolin;

mit 2-(Pyrrolidino-methyl)-pyrrolidin:

(2S)-1-Bis-(p-fluor-phenyl)-acetyl-2-(pyrrolidino-methyl)-pyrrolidin;

mit N-Methyl-N-(2-(pyrrolidino-4-methyl-pentyl)-amin:
N-Methyl-N-(2-(pyrrolidino-4-methyl-pentyl)-2,2-bis-(p-fluor-phenyl)-acetamid;

mit N-Methyl-N-[2-((3S)-hydroxy-pyrrolidino)-4-methyl-pentyl]-amin:
N-Methyl-N-[2-((3S)-hydroxy-pyrrolidino)-4-methyl-pentyl]-2,2-bis-(p-fluor-phenyl)-acetamid;

mit N-Methyl-N-(2-pyrrolidino-3-methyl-butyl)-amin:
N-Methyl-N-(2-pyrrolidino-3-methyl-butyl)-2,2-bis-(p-fluor-phenyl)-acetamid;

mit N-Methyl-N-[2-((3S)-hydroxy-pyrrolidino)-3-methyl-butyl]-amin:
N-Methyl-N-[2-((3S)-hydroxy-pyrrolidino)-3-methyl-butyl]-2,2-bis-(p-fluor-phenyl)-acetamid;

mit (1S)-1-Methyl-amino-1-(p-hydroxy-phenyl)-2-((3S)-3-hydroxy-pyrrolidino)-ethan:
N-Methyl-N-[(1S)-1-(p-hydroxy-phenyl)-2-((3S)-3-hydroxy-pyrrolidino)-ethyl]-2,2-bis-(p-fluor-phenyl)-acetamid;

mit (1S)-1-Methyl-amino-1-(p-chlor-phenyl)-2-pyrrolidino-ethan:
N-Methyl-N-[(1S)-1-(p-chlor-phenyl)-2-pyrrolidino-ethyl]-2,2-bis-(p-fluor-phenyl)-acetamid;

mit 1-((3S)-3-Hydroxy-pyrrolidino-methyl)-1,2,3,4-tetrahydroisochinolin:
2-[Bis-(p-fluor-phenyl)-acetyl]-1-((3S)-3-hydroxy-pyrrolidino-methyl)-1,2,3,4-tetrahydro-isochinolin;

mit (1S)-1-Methyl-amino-1-(2,4-di-methoxy-phenyl)-2-((3S)-3-hydroxy-pyrrolidino)-ethan:
N-Methyl-N-[1S)-1-(2,4-di-methoxy-phenyl)-2-((3S)-3-hydroxy-pyrrolidino)-ethyl]-2,2-bis-(p-fluor-phenyl)-acetamid;

mit (1S)-1-Methyl-amino-1-(2,4-di-methoxy-phenyl)-2-pyrrolidino-ethan:
N-Methyl-N-[(1S)-1-(2,4-di-methoxy-phenyl)-2-pyrrolidino-ethyl]-2,2-bis-(p-fluor-phenyl)-acetamid;

mit (1S)-1-Methyl-amino-1-(p-trifluormethyl-phenyl)-2-((3S)-3-hydroxy-pyrrolidino)-ethan:
N-Methyl-N-[(1S))-1-(p-trifluormethyl-phenyl)-2-((3S)-3-hydroxy-pyrrolidino)-ethyl]-2,2-bis-(p-fluor-phenyl)-acetamid;

mit (1S)-1-Methyl-amino-1-p-methyl-phenyl-2-((3S)-3-hydroxy-pyrrolidino)-ethan:
N-Methyl-N-[(1S)-1-(p-methyl-phenyl)-2-((3S)-3-hydroxy-pyrrolidino)-ethyl]-2,2-bis-(p-fluor-phenyl)-acetamid;

mit (1S)-1-Methyl-amino-1-(p-amino-phenyl)-2-((3S)-3-hydroxy-pyrrolidino)-ethan:
N-Methyl-N-[(1S)-1-(p-amino-phenyl)-2-((3S)-3-hydroxy-pyrrolidino)-ethyl]-2,2-bis-(p-fluor-phenyl)-acetamid;

mit (1S)-1-Methyl-amino-1-(p-fluor-phenyl)-2-((3S)-3-hydroxy-pyrrolidino)-ethan:
N-Methyl-N-[(1S)-1-(p-fluor-phenyl)-2-((3S)-3-hydroxy-pyrrolidino)-ethyl]-2,2-bis-(p-fluor-phenyl)-acetamid;

mit (1S)-1-Methyl-aminol-(2,4-di-fluor-phenyl)-2-((3S)-3-hydroxy-pyrrolidino)-ethan:
N-Methyl-N-[(1S)-1-(2,4-di-fluor-phenyl)-2-((3S)-3-hydroxy-pyrrolidino)-ethyl]-2,2-bis-(p-fluor-phenyl)-acetamid.

Beispiel 3

Analog Beispiel 1 erhält man durch Umsetzung von Bis-(p-chlor-phenyl)-essigsäure

mit (1S)-1-Methyl-amino-1-phenyl-2-((3S)-3-hydroxy-pyrrolidino)-ethan:
N-Methyl-N-[(1S)-1-phenyl-2-((3S)-3-hydroxy-pyrrolidino)-ethyl]-2,2-bis-(p-chlor-phenyl)-acetamid;

mit (1S)-1-Methyl-amino-1-(p-methoxy-phenyl)-2-((3S)-3-hydroxy-pyrrolidino)-ethan:

N-Methyl-N-[(1S)-1-(p-methoxy-phenyl)-2-((3S)-3-hydroxy-pyrrolidino)-ethyl]-2,2-bis-(p-chlor-phenyl)-acetamid;

mit (1S)-1-Methyl-amino-1-phenyl-2-pyrrolidino-ethan:
N-Methyl-N-[(1S)-1-phenyl-2-pyrrolidino-ethyl]-2,2-bis-(p-chlor-phenyl)-acetamid;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-bis-(p-chlor-phenyl)-acetamid;

mit 1-(Pyrrolidino-methyl)-1,2,3,4-tetrahydroisochinolin:
2-[Bis-(p-chlor-phenyl)-acetyl]-1-(pyrrolidino-methyl)-1,2,3,4-tetrahydro-isochinolin;

mit 2-(Pyrrolidino-methyl)-pyrrolidin;
(2S)-1-Bis-(p-chlor-phenyl)-acetyl-2-(pyrrolidino-methyl)-pyrrolidin;

mit N-Methyl-N-(2-pyrrolidino-4-methyl-pentyl)-amin:
N-Methyl-N-(2-pyrrolidino-4-methyl-pentyl)-2,2-bis-(p-chlor-phenyl)-acetamid;

mit N-Methyl-N-[2-((3S)-hydroxy-pyrrolidino)-4-methyl-pentyl]-amin:
N-Methyl-N-[2-((3S)-hydroxy-pyrrolidino)-4-methyl-pentyl]-2,2-bis-(p-chlor-phenyl)--acetamid;

mit N-Methyl-N-[2-pyrrolidino-methyl-butyl)-amin:
N-Methyl-N-(2-pyrrolidino-3-methyl-butyl)-2,2-bis-(p-chlor-phenyl)-acetamid;

mit N-Methyl-N-[2-((3S)-hydroxy-pyrrolidino)-3-methyl-butyl]-amin:
N-Methyl-N-[2-((3S)-hydroxy-pyrrolidino)-3-methyl-butyl]-2,2-bis-(p-chlor-phenyl)-acetamid;

mit (1S)-1-Methyl-amino-1-(p-hydroxy-phenyl)-2-((3S)-3-hydroxy-pyrrolidino)-ethan:
N-Methyl-N-[(1S)-1-(p-hydroxy-phenyl)-2-((3S)-3-hydroxy-pyrrolidino)-ethyl]-2,2-bis-(p-chlor-phenyl)-acetamid;

mit (1S)-1-Methyl-amino-1-(p-chlor-phenyl)-2-pyrrolidino-ethan:
N-Methyl-N-[(1S)-1-(p-chlor-phenyl)-2-pyrrolidino-ethyl]-2,2-bis-(p-chlor-phenyl)-acetamid;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-bis-(p-chlor-phenyl)-acetamid;

mit 1-((3S)-3-Hydroxy-pyrrolidino-methyl)-1,2,3,4-tetrahydroisochinolin:
2-[Bis-(p-chlor-phenyl)-acetyl]-1-((3S)-3-hydroxy-pyrrolidino-methyl)-1,2,3,4-tetrahydro-isochinolin;

mit (1S)-1-Methyl-amino-1-(2,4-di-methoxy-phenyl)-2-((3S)-3-hydroxy-pyrrolidino)-ethan:
N-Methyl-N-[(1S)-1-(2,4-di-methoxy-phenyl)-2-((3S)-3-hydroxy-pyrrolidino)-ethyl]-2,2-bis-(p-chlor-phenyl)-acetamid;

mit (1S)-1-Methyl-amino-1-(2,4-di-methoxy-phenyl)-2-pyrrolidino-ethan:
N-Methyl-N-[(1S)-1-(2,4-di-methoxy-phenyl)-2-pyrrolidino-ethyl]-2,2-bis-(p-chlor-phenyl)-acetamid;

mit (1S)-1-Methyl-aminol-1-(p-trifluormethyl-phenyl)-2-((3S)-3-hydroxy-pyrrolidino)-ethan:
N-Methyl-N-[(1S)-1-(p-trifluormethyl-phenyl)-2-((3S)-3-hydroxy-pyrrolidino)-ethyl]-2,2-bis-(p-chlor-phenyl)-acetamid;

mit (1S)-1-Methyl-amino-1-(p-methyl-phenyl)-2-((3S)-3-hydroxy-pyrrolidino)-ethan:
N-Methyl-N-[(1S)-1-(p-methyl-phenyl)-2-((3S)-3-hydroxy-pyrrolidino)-ethyl]-2,2-bis-(p-chlor-phenyl)-acetamid;

mit (1S)-1-Methyl-amino-1-(p-amino-phenyl)-2-((3S)-3-hydroxy-pyrrolidino)-ethan:

N-Methyl-N-[(1S)-1-(p-amino-phenyl)-2-((3S)-3-hydroxy-pyrrolidino)-ethyl]-2,2-bis-(p-chlor-phenyl)-acetamid;

mit (1S)-1-Methyl-amino-1-(p-chlor-phenyl)-2-((3S)-3-hydroxy-pyrrolidino)-ethan:
N-Methyl-N-[(1S)-1-(p-chlor-phenyl)-2-((3S)-3-hydroxy-pyrrolidino)-ethyl-2,2-bis-(p-chlor-phenyl)-acetamid;

mit (1S)-1-Methyl-amino-1-(2,4-di-fluor-phenyl)-2-((3S)-3-hydroxy-pyrrolidino)-ethan:
N-Methyl-N-[(1S)-1-(2,4-di-fluor-phenyl)-2-((3S)-3-hydroxy-pyrrolidino)-ethyl]-2,2-bis-(p-chlor-phenyl)-acetamid.

Beispiel 4

Eine Lösung von 4,6 g (1S)-1-Methyl-amino-1-phenyl-2-((3S)-3-hydroxy-pyrrolidino)-ethan [erhältlich aus (1S)-1-Amino-1-phenyl-2-chlorethan durch Umsetzung mit (3S)-3-Hydroxypyrrolidin und anschließende Methylierung mit Methyliodid] in 200 ml Dichlormethan wird mit 30 ml Trimethylamin-Lösung (33%ig) versetzt. Anschließend tropft man unter Rühren eine Lösung von 1 Äquivalent 9-Fluoren-carbonsäurechlorid in 200 ml Dichlormethan hinzu, rührt 2Std. bei Raumtemperatur und erhält nach üblicher Aufarbeitung N-Methyl-N-[(1S)-1-phenyl-2-((3S)-3-hydroxy-pyrrolidino)-ethyl]-9-fluoren-carboxamid, Rf: 0,61.

Analog erhält man durch Umsetzung von 9-Fluoren-carbonsäure-chlorid

mit (1S)-1-Methyl-amino-1-phenyl-2-pyrrolidino]-ethan:
N-Methyl-N-[(1S)-1-phenyl-2-pyrrolidino-ethyl]-9-fluoren-carboxamid, Rf: 0,67;

mit 1-(Pyrrolidino-methyl)-1,2,3,4-tetrahydroisochinolin:
2-(9-Fluoren-carbonyl)-1-(pyrrolidinomethyl)-1,2,3,4-tetrahydroisochinolin, Rf: 0,77;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-9-fluorencarboxamid;

mit (1S)-1-Methyl-amino-1-(p-methyl-phenyl)-2-pyrrolidino-ethan:
N-Methyl-N-[(1S)-1-(p-methyl-phenyl)-2-pyrrolidino-ethyl]-9-fluorencarboxamid;

mit (1S)-1-Methyl-amino-1-(p-ethoxy-phenyl)-2-((3S)-3-hydroxy-pyrrolidino)-ethan:
N-Methyl-N-[(1S)-1-(p-ethoxy-phenyl)-2-((3S)-3-hydroxy-pyrrolidino)-ethyl]-9-fluorencarboxamid;

mit 1-[(3S)-3-Hydroxy-pyrrolidino-methyl]-1,2,3,4-tetrahydroisochinolin:
2-(9-Fluoren-carbonyl)-1-[(3S)-3-Hydroxy-pyrrolidino-methyl]-1,2,3,4-tetrahydroisochinolin;

mit (2S)-2-(Pyrrolidino-methyl)-pyrrolidin:
(2S)-1-(9-Fluoren-carbonyl)-2-(pyrrolidino-methyl)-pyrrolidin;

mit N-Methyl-N-(2-(pyrrolidino-4-methyl-pentyl)-amin:
N-Methyl-N-(2-(pyrrolidino-4-methyl-pentyl)-9-fluoren-carboxamid;

mit N-Methyl-N-[2-((3S)-hydroxy-pyrrolidino)-4-methyl-pentyl]-amin:
N-Methyl-N-[2-((3S)-hydroxy-pyrrolidino)-4-methyl-pentyl]-9-fluorencarboxamid;

mit N-Methyl-N-(2-(pyrrolidino-3-methyl-butyl-amin:
N-Methyl-N-[2-(1-pyrrolidino-3-methyl)-butyl]-9-fluoren-carboxamid;

mit N-Methyl-N-(2-((3S)-hydroxy-pyrrolidino)-3-methyl-butyl)-amin:
N-Methyl-N-(2-((3S)-hydroxy-pyrrolidino)-3-methyl-butyl]-9-fluorencarboxamid;

mit (1S)-1-Methyl-amino-1-(p-hydroxy-phenyl)-2-((3S)-3-hydroxy-pyrrolidino)-ethan:
N-Methyl-N-[(1S)-1-(p-hydroxy-phenyl)-2-((3S)-3-hydroxy-pyrrolidino)-ethyl1-9-fluorencarboxamid;

mit (1S)-1-Methyl-amino-1-(p-chlor-phenyl)-2-pyrrolidino-ethan:

N-Methyl-N-[(1S)-1-(p-chlor-phenyl)-2-pyrrolidino-ethyl]-9-fluorencarboxamid;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-9-fluorencarboxamid;

mit (1S)-1-Methyl-amino-1-(2,4-di-methoxy-phenyl)-2-((3S)-3-hydroxy-pyrrolidino)-ethan:
N-Methyl-N-[(1S)-1-(2,4-di-methoxy-phenyl)-2-((3S)-3-hydroxy-pyrrolidino)-ethyl]-9-fluorencarboxamid;

mit (1S)-1-Methyl-amino-1-(2,4-di-methoxy-phenyl)-2-pyrrolidino-ethan:
N-Methyl-N-[(1S)-1-(2,4-di-methoxy-phenyl)-2-pyrrolidino-ethyl]-9-fluorencarboxamid;

mit (1S)-1-Methyl-amino-1-(p-trifluormethyl-phenyl)-2-((3S)-3-hydroxy-pyrrolidino)-ethan:
N-Methyl-N-[(1S)-1-(p-trifluormethyl-phenyl)-2-((3S)-3-hydroxy-pyrrolidino)-ethyl]-9-fluorencarboxamid;

mit (1S)-1-Methyl-amino-1-(p-methyl-phenyl)-2-((3S)-3-hydroxy-pyrrolidino)-ethan:
N-Methyl-N-[(1S)-1-(p-methyl-phenyl)-2-((3S)-3-hydroxy-pyrrolidino)-ethyl]-9-fluorencarboxamid;

mit (1S)-1-Methyl-amino-1-(p-amino-phenyl)-2-((3S)-3-hydroxy-pyrrolidino)-ethan:
N-Methyl-N-[(1S)-1-(p-amino-phenyl)-2-((3S)-3-hydroxy-pyrrolidino)-ethyl]-9-fluorencarboxamid;

mit (1S)-1-Methyl-amino-1-(p-fluor-phenyl)-2-((3S)-3-hydroxy-pyrrolidino)-ethan:
N-Methyl-N-[(1S)-1-(p-fluor-phenyl)-2-((3S)-3-hydroxy-pyrrolidino)-ethyl]-9-fluorencarboxamid;

mit (1S)-1-Methyl-amino-1-(2,4-di-fluor-phenyl)-2-((3S)-3-hydroxy-pyrrolidino)-ethan:
N-Methyl-N-[(1S)-1-(2,4-di-fluor-phenyl)-2-((3S)-3-hydroxy-pyrrolidino)-ethyl-9-fluorencarboxamid.

Beispiel 5

Analog Beispiel 4 erhält man durch Umsetzung von 9-Xanthencarbonsäure-chlorid

mit (1S)-1-Methyl-amino-1-phenyl-2-pyrrolidino-ethan:
N-Methyl-N-[(1S)-1-phenyl-2-pyrrolidino-ethyl]-9-xanthen-carboxamid, Rf: 0,77;

mit (1S)-1-Methyl-amino-1-phenyl-2-((3S)-3-hydroxy-pyrrolidino)-ethan:
N-Methyl-N-[(1S)-1-phenyl-2-((3S)-3-hydroxy-pyrrolidino)-ethyl]-9-xanthencarboxamid, Rf: 0,64;

mit 1-(Pyrrolidino-methyl)-1,2,3,4-tetrahydroisochinolin:
2-(9-Xanthen-carbonyl)-1-(pyrrolidinomethyl)-1,2,3,4-tetrahydroisochinolin;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-9-xanthencarboxamid;

mit (1S)-1-Methyl-amino-1-(p-methyl-phenyl)-2-pyrrolidino-ethan:
N-Methyl-N-[(1S)-1-(p-methyl-phenyl)-2-pyrrolidino-ethyl]-9-xanthencarboxamid;

mit (1S)-1-Methyl-amino-1-(p-ethoxy-phenyl)-2-((3S)-3-hydroxy-pyrrolidino)-ethan:
N-Methyl-N-[(1S)-1-(p-ethoxy-phenyl)-2-((3S)-3-hydroxy-pyrrolidino)-ethyl]-9-xanthencarboxamid;

mit 1-[(3S)-3-Hydroxy-pyrrolidino-methyl]-1,2,3,4-tetrahydro-isochinolin:
2-(9-Xanthen-carbonyl)-1-[(3S)-3-hydroxy-pyrrolidino-methyl]-1,2,3,4-tetrahydroisochinolin;

mit 1-(Pyrrolidino-methyl)-1,2,3,4-tetrahydro-isochinolin:
2-(9-Xanthen-carbonyl)-1-(pyrrolidino-methyl)-1,2,3,4-tetrahydroisochinolin, Rf: 0,76;

mit (2S)-2-(Pyrrolidino-methyl)-pyrrolidin:
(2S)-1-(9-Xanthen-carbonyl)-2-(pyrrolidino-methyl)-pyrrolidin;

mit N-Methyl-N-(2-pyrrolidino-4-methyl-pentyl)-amin;
N-Methyl-N-(2-(pyrrolidino-4-methyl)-pentyl)-9-xanthen-carboxamid;

mit N-Methyl-N-[2-((3S)-hydroxy-pyrrolidino)-4-methyl-pentyl]-amin:
N-Methyl-N-[2-((3S)-hydroxy-pyrrolidino)-4-methyl-pentyl]-9-xanthencarboxamid;

mit N-Methyl-N-(2-pyrrolidino-3-methyl-butyl)-amin:
M-Methyl-N-(2-(pyrrolidino-3-methyl-butyl)-9-xanthen-carboxamid;

mit N-Methyl-N-[2-((3S)-hydroxy-pyrrolidino)-3-methyl-butyl]-amin:
N-Methyl-N-[2-((3S)-hydroxy-pyrrolidino)-3-methyl-butyl]-9-xanthencarboxamid;

mit (1S)-1-Methyl-amino-1-(p-hydroxy-phenyl)-2-((3S)-3-hydroxy-pyrrolidino)-ethan:
N-Methyl-N-[(1S)-1-(p-hydroxy-phenyl)-2-((3S)-3-hydroxy-pyrrolidino)-ethyl]-9-xanthencarboxamid;

mit (1S)-1-Methyl-amino-1-(p-chlor-phenyl)-2-pyrrolidino]-ethan:
N-Methyl-N-[(1S)-1-(p-chlor-phenyl)-2-pyrrolidino-ethyl]-9-xanthencarboxamid;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-9-xanthencarboxamid;

mit (1S)-1-Methyl-amino-1-(2,4-di-methoxy-phenyl)-2-((3S)-3-hydroxy-pyrrolidino)-ethan:
N-Methyl-N-[(1S)-1-(2,4-di-methoxy-phenyl)-2-((3S)-3-hydroxy-pyrrolidino)-ethyl]-9-xanthencarboxamid;

mit (1S)-1-Methyl-amino-1-(2,4-di-methoxy-phenyl)-2-pyrrolidino-ethan:
N-Methyl-N-[(1S)-1-(2,4-di-methoxy-phenyl)-2-pyrrolidino-ethyl]-9-xanthencarboxamid;

mit (1S)-1-Methyl-amino-1-(p-trifluormethyl-phenyl)-2-((3S)-3-hydroxy-pyrrolidino)-ethan:
N-Methyl-N-[(1S)-1-(p-trifluormethyl-phenyl)-2-((3S)-3-hydroxy-pyrrolidino)-ethyl]-9-xanthencarboxamid;

mit (1S)-1-Methyl-amino-1-(p-methyl-phenyl)-2-((3S)-3-hydroxy-pyrrolidino)-ethan:
N-Methyl-N-[(1S)-1-(p-methyl-phenyl)-2-((3S)-3-hydroxy-pyrrolidino)-ethyl]-9-xanthencarboxamid;

mit (1S)-1-Methyl-amino-1-(p-amino-phenyl)-2-((3S)-3-hydroxy-pyrrolidino)-ethan:
N-Methyl-N-[(1S)-1-(p-amino-phenyl)-2-((3S)-3-hydroxy-pyrrolidino)-ethyl]-9-xanthencarboxamid;

mit (1S)-1-Methyl-amino-1-(p-fluor-phenyl)-2-((3S)-3-hydroxy-pyrrolidino)-ethan:
N-Methyl-N-[(1S)-1-(p-fluor-phenyl)-2-((3S)-3-hydroxy-pyrrolidino)-ethyl]-9-xanthencarboxamid;

mit (1S)-1-Methyl-amino-1-(2,4-di-fluor-phenyl)-2-((3S)-3-hydroxy-pyrrolidino)-ethan:
N-Methyl-N-[(1S)-1-(2,4-di-fluor-phenyl)-2-((3S)-3-hydroxy-pyrrolidino)-ethyl]-9-xanthencarboxamid.

<u>Beispiel 6</u>

Analog Beispiel 1 erhält man durch Umsetzung von 2,2-Di-phenyl-propionsäurechlorid

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-diphenylpropionamid;

mit (1S)-1-Methyl-amino-1-phenyl-2-pyrrolidino-ethan:
N-Methyl-N-[(1S)-1-phenyl-2-pyrrolidino-ethyl]-2,2-diphenylpropionamid;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-diphenylpropionamid;

mit 1-(Pyrrolidino-methyl)-1,2,3,4-tetrahydroisochinolin:
2-(2,2-Diphenylpropionyl)-1-(pyrrolidino-methyl)-1,2,3,4-tetrahydroisochinolin, Rf: 0,65;

mit 2-(Pyrrolidino-methyl)-pyrrolidin:
(2S)-1-(2,2-Diphenylpropionyl)-2-(pyrrolidino-methyl)-pyrrolidin;

mit N-Methyl-N-(2-(pyrrolidino-4-methyl-pentyl)-amin;
N-Methyl-N-(2-(pyrrolidino-4-methyl-pentyl)-2,2-diphenyl-propionamid;

mit N-Methyl-N-[2-(1-((3S)-hydroxy-pyrrolidino)-4-methyl)-pentyl]-amin:
N-Methyl-N-[2-((3S)-hydroxy-pyrrolidino)-4-methyl-pentyl]-2,2-diphenyl-propionamid;

mit N-Methyl-N-(2-(pyrrolidino-3-methyl-butyl)-amin:
N-Methyl-N-2-(pyrrolidino-3-methyl)-butyl)-2,2-diphenyl-propionamid;

mit N-Methyl-N-[2-((3S)-hydroxy-pyrrolidino)-3-methyl-butyl]-amin:
N-Methyl-N-[2-((3S)-hydroxy-pyrrolidino)-3-methyl-butyl]-2,2-diphenyl-propionamid;

mit (1S -1-Methyl-amino-1-(p-hydroxy-phenyl)-2-((3S)-3-hydroxy-pyrrolidino)-ethan:
N-Methyl-N-[(1S)-1-(p-hydroxy-phenyl)-2-((3S)-3-hydroxy-pyrrolidino)-ethyl]-2,2-diphenylpropionamid;

mit (1S)-1-Methyl-amino-1-(p-chlor-phenyl)-2-pyrrolidino-ethan:
N-Methyl-N-[(1S)-1-(p-chlor-phenyl)-2-pyrrolidino-ethyl]-2,2-diphenylpropionamid

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-diphenylpropionamid;

mit 1-((3S)-3-Hydroxy-pyrrolidino-methyl)-1,2,3,4-tetrahydroisochinolin:
2-(2,2-Diphenylpropionyl)-1-((3S)-3-hydroxy-pyrrolidino-methyl)-1,2,3,4-tetrahydro-isochinolin;

mit (1S)-1-Methyl-amino-1-(2,4-di-methoxy-phenyl)-2-((3S)-3-hydroxy-pyrrolidino)-ethan:
N-Methyl-N-[(1S)-1-(2,4-di-methoxy-phenyl)-2-((3S)-3-hydroxy-pyrrolidino)-ethyl)-2,2-diphenylpropionamid;

mit (1S)-1-Methyl-amino-1-(2,4-di-methoxy-phenyl)-2-pyrrolidino-ethan:
N-Methyl-N-[(1S)-1-(2,4-di-methoxy-phenyl)-2-pyrrolidino-ethyl]-2,2-diphenylpropionamid;

mit (1S)-1-Methyl-amino-1-(p-trifluormethyl-phenyl)-2-((3S)-3-hydroxy-pyrrolidino)-ethan:
N-Methyl-N-[(1S)-1-(p-trifluormethyl-phenyl)-2-((3S)-3-hydroxy-pyrrolidino)-ethyl]-2,2-diphenylpropionamid;

mit (1S)-1-Methyl-amino-1-(p-methyl-phenyl)-2-((3S)-3-hydroxy-pyrrolidino)-ethan:
N-Methyl-N-[(1S)-1-(p-methyl-phenyl)-2-((3S)-3-hydroxy-pyrrolidino)-ethyl]-2,2-diphenylpropionamid;

mit (1S)-1-Methyl-amino-1-(p-amino-phenyl)-2-((3S)-3-hydroxy-pyrrolidino)-ethan:
N-Methyl-N-[(1S)-1-(p-amino-phenyl)-2-((3S)-3-hydroxy-pyrrolidino)-ethyl]-2,2-diphenylpropionamid;

mit (1S)-1-Methyl-amino-1-(p-fluor-phenyl)-2-((3S)-3-hydroxy-pyrrolidino)-ethan:
N-Methyl-N-[(1S)-1-(p-fluor-phenyl)-2-((3S)-3-hydroxy-pyrrolidino)-ethyl]-2,2-diphenylpropionamid;

mit (1S)-1-Methyl-amino-1-(2,4-di-fluor-phenyl)-2-((3S)-3-hydroxy-pyrrolidino)-ethan:
N-Methyl-N-[(1S)-1-(2,4-di-fluor-phenyl)-2-((3S)-3-hydroxy-pyrrolidino)-ethyl]-2,2-diphenylpropionamid.

Beispiel 7

Analog Beispiel 1 erhält man durch Umsetzung von 2,2-Bis-(p-fluor-phenyl)-propionsäure

mit (1S)-1-Methyl-amino-1-phenyl-2-((3S)-3-hydroxy-pyrrolidino)-ethan:
N-Methyl-N-[(1S)-1-phenyl-2-((3S)-3-hydroxy-pyrrolidino)-ethyl]-2,2-bis-(p-fluor-phenyl)-propionamid;

mit (1S)-1-Methyl-amino-1-(p-methoxy-phenyl)-2-((3S)-3-hydroxy-pyrrolidino)-ethan:

N-Methyl-N-[(1S)-1-(p-methoxy-phenyl)-2-((3S)-3-hydroxy-pyrrolidino)-ethyl]-2,2-bis-(p-fluor-phenyl)-propiomamid;

mit (1S)-1-Methyl-amino-1-pheny-2-pyrrolidino-ethan:
N-Methyl-N-[(1S)-1-phenyl-2-pyrrolidino-ethyl]-2,2-bis-(p-fluor-phenyl)-propionamid;

mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-bis-(p-fluor-phenyl)-propionamid;

mit 1-(Pyrrolidino-methyl)-1,2,3,4-tetrahydroisochinolin:
2-[2,2-Bis-(p-fluor-phenyl)-propionyl]-1-(pyrrolidino-methyl)-1,2,3,4-tetrahydro-isochinolin;

mit N-Methyl-N-(2-(pyrrolidino-4-methyl-pentyl)-amin:
N-Methyl-N-2-(pyrrolidino-4-methyl-pentyl)-2,2-bis-(p-fluor-phenyl)-propionamid;

mit N-Methyl-N-[2-((3S)-hydroxy-pyrrolidino)-4-methyl-pentyl]-amin:
N-Methyl-N-[2-((3S)-hydroxy-pyrrolidino)-4-methyl-pentyl]-2,2-bis-(p-fluor-phenyl)-propionamid;

mit N-Methyl-N-[2-((3S)-hydroxy-pyrrolidino)-3-methyl-butyl]-amin:
N-Methyl-N-[2-((3S)-hydroxy-pyrrolidino)-3-methyl-butyl]-2,2-bis-(p-fluor-phenyl)--propionamid;

mit (1S)-1-Methyl-amino-1-(p-fluor-phenyl)-2-((3S)-3-hydroxy-pyrrolidino)-ethan:
N-Methyl-N-[(1S)-1-(p-fluor-phenyl)-2-((3S)-3-hydroxy-pyrrolidino)-ethyl]-2,2-bis-(p-fluor-phenyl)-propionamid;

mit (1S)-1-Methyl-amino-1-(2,4-di-fluor-phenyl)-2-((3S)-3-hydroxy-pyrrolidino)-ethan:
N-Methyl-N [(1S)-1-(2,4-di-fluor-phenyl)-2-((3S)-3-hydroxy-pyrrolidino)-ethyl]-2,2-bis-(p-fluor-phenyl)-propionamid.

<u>Beispiel 8</u>

Analog Beispiel 1 erhält man durch Umsetzung von 2,2-Bis-(p-chlor-phenyl) propionylchlorid

mit (1S)-1-Methyl-amino-1-phenyl-2-((3S)-3-hydroxy-pyrrolidino)-ethan:
N-Methyl-N-[(1S)-1-phenyl-2-((3S)-3-hydroxy-pyrrolidino)-ethyl]-2,2-di-(p-chlor-phenyl)-propionamid;
mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-di-(p-chlor-phenyl)-propionamid;
mit (1S)-1-Methyl-amino-1-phenyl-2-pyrrolidino-ethan:
N-Methyl-N-[(1S)-1-phenyl-2-pyrrolidino-ethyl]-2,2-di-(p-chlor-phenyl)-propionamid;
mit (1S)-1-Methyl-amino-1-(p-methoxy-phenyl)-2-((3S)-3-hydroxy-pyrrolidino)-ethan:
N-Methyl-N-[(1S)-1-(p-methoxy-phenyl)-2-((3S)-3-hydroxy-pyrrolidino)-ethyl]-2,2-di-(p-chlor-phenyl)-propionamid;
mit N-Methyl-N-[2-((3S)-hydroxy-pyrrolidino)-3-methyl-butyl]-amin:
N-Methyl-N-[2-((3S)-hydroxy-pyrrolidino)-3-methyl-butyl]-2,2-bis(p-chlor-phenyl)--propionamid;
mit (1S)-1-Methyl-amino-1-(p-hydroxy-phenyl)-2-((3S)-3-hydroxy-pyrrolidino) -ethan:
N-Methyl-N-[(1S)-1-(p-hydroxy-phenyl)-2-((3S)-3-hydroxy-pyrrolidino)-ethyl]-2,2-bis-(p-chlor-phenyl)-propionamid;
mit (1S)-1-Methyl-amino-1-(p-amino-phenyl)-2-((3S)-3-hydroxy-pyrrolidino)-ethan:
N-Methyl-N-[(1S)-1-(p-amino-phenyl)-2-((3S)-3-hydroxy-pyrrolidino)-ethyl]-2,2-bis-(p-chlor-phenyl)-propionamid;
mit (1S)-1-Methyl-amino-1-(p-chlor-phenyl)-2-((3S)-3-hydroxy-pyrrolidino)-ethan:
N-Methyl-N-[(1S)-1-(p-chlor-phenyl)-2-((3S)-3-hydroxy-pyrrolidino)-ethyl]-2,2-bis-(p-chlor-phenyl)-propionamid;
mit (1S)-1-Methyl-amino-1-(2,4-di-fluor-phenyl)-2-((3S)-3-hydroxy-pyrrolidino)-ethan:
N-Methyl-N-[(1S)-1-(2,4-di-fluor-phenyl)-2-((3S)-3-hydroxy-pyrrolidino)-ethyl]-2,2-bis-(p-chlor-phenyl)-propionamid.

Beispiel 9

Man löst 4,3 g Bis-(p-chlorphenyl)-essigsäurehydrazid [z.B. erhältlich aus dem entsprechenden Ethylester durch Umsetzung mit Hydrazin] in 200 ml stark verdünnter Salzsäure, tropft unter Rühren bei 0° eine Lösung von 2,0 g $NaNO_2$ in 40 ml Wasser hinzu, rührt 30 Min. und extrahiert das gebildete Azid mit Dichlormethan. Nach Trocknen über $MgSO_4$ und Konzentrieren auf 50 ml wird das so erhaltene Reagenz zu einer Lösung von (1S)-1-Methyl-amino-1-(p-methoxy-phenyl)-2-((3S)-3-hydroxy-pyrrolidino)-ethan und 4 ml Triethylamin in 100 ml Dichlormethan zugetropft. Man rührt noch 2 Std. bei 20° und erhält nach üblicher Aufarbeitung N-Methyl-N-[(1S)-1-(p-methoxy-phenyl)-2-((3S)-3-hydroxy-pyrrolidino)-ethyl]-2,2-bis-(p-chlor-phenyl)-acetamid.

Beispiel 10

Man hydriert eine Lösung von 1 g N-Methyl-N-[(1S)-1-(p-benzyloxy-phenyl)-2-((3S)-3-hydroxy-pyrrolidino)-ethyl]-2,2-diphenyl-acetamid in 25 ml Ethylacetat an 0,5 g 5%iger Pd-C bei 20° und 1 bar bis zum Stillstand der Wasserstoffaufnahme, filtriert, dampft ein und erhält N-Methyl-N-[(1S)-1-(p-hydroxy-phenyl)-2-((3S)-3-hydroxy-pyrrolidino)-ethyl]-2,2-di-phenyl-acetamid.

Analog erhält man aus den entsprechenden o- und m-Benzyloxy-derivaten:

N-Methyl-N-[(1S)-1-(o-hydroxy-phenyl)-2-((3S)-3-hydroxy-pyrrolidino)-ethyl-2,2-diphenyl-acetamid;
N-Methyl-N-[(1S)-1-(m-hydroxy-phenyl)-2-((3S)-3-hydroxy-pyrrolidino)-ethyl]-2,2-diphenyl-acetamid;
N-Methyl-N-[(1S)-1-(o-hydroxy-phenyl)-2-pyrrolidino-ethyl]-2,2-diphenyl-acetamid;
N-Methyl-N-[(1S)-1-(m-hydroxy-phenyl)-2-pyrrolidino-ethyl]-2,2-diphenyl-acetamid;
N-Methyl-N-[(1S)-1-(o-hydroxy-phenyl)-2-pyrrolidino-ethyl]-2,2-bis-(p-chlor-phenyl)-acetamid;
N-Methyl-N-[(1S)-1-(m-hydroxy-phenyl)-2-pyrrolidino-ethyl)-2,2-bis-(p-chlor-phenyl)-acetamid;
N-Methyl-N-[(1S)-1-(o-hydroxy-phenyl)-2-pyrrolidino-ethyl]-2,2-bis-(p-fluor-phenyl)-acetamid;
N-Methyl-N-[(1S)-1-(m-hydroxy-phenyl)-2-pyrrolidino-ethyl]-2,2-bis-(p-fluor-phenyl)-acetamid.

Analog erhält man aus den entsprechenden p-Benzyloxy-derivaten:

N-Methyl-N-[(1S)-1-(p-hydroxy-phenyl)-2-pyrrolidino-ethyl]-2,2-diphenyl-acetamid;
N-Methyl-N-[(1S)-1-(p-hydroxy-phenyl)-2-pyrrolidino-ethyl]-2,2-bis-(p-chlor-phenyl)-acetamid;
N-Methyl-N-[(1S)-1-(p-hydroxy-phenyl)-2-pyrrolidino-ethyl]-2,2-bis-(p-fluor-phenyl)-acetamid.

Beispiel 11

Man löst 3,2 g N-[(1S)-1-phenyl-2-((3S)-3-hydroxy-pyrrolidino)-ethyl]-2,2-diphenylacetamid in 150 ml Dichlormethan tropft unter Rühren 1 Äquivalent Methyliodid, gelöst in 10 ml Dichlormethan, hinzu, konzentriert die Lösung und erhält nach üblicher Aufarbeitung N-Methyl-N-[(1S)-1-phenyl-2-((3S)-3-hydroxy-pyrrolidino)-ethyl]-2,2-diphenylacetamid, Rf: 0,60.

Analog erhält man durch Umsetzung mit Methyliodid

aus
N-[(1S)-1-(p-methoxy-phenyl)-2-((3S)-3-hydroxy-pyrrolidino)-ethyl]-2,2-diphenylacetamid: N-Methyl-N-[(1S)-1-(p-methoxy-phenyl)-2-((3S)-3-hydroxy-pyrrolidino)-ethyl]-2,2-diphenylacetamid, Rf: 0,61;

aus
N-[(1S)-1-phenyl-2-pyrrolidino-ethyl]-2,2-diphenylacetamid: N-Methyl-N-[(1S)-1-phenyl-2-pyrrolidino-ethyl]-2,2-diphenylacetamid, Rf: 0,71;

aus
N-[(1S)-1-(p-methoxy-phenyl)-2-((3S)-3-hydroxy-pyrrolidino)ethyl]-2,2-diphenylacetamid: N-Methyl-N-[(1S)-1-(p-methoxy-phenyl)-2-((3S)-3-hydroxy-pyrrolidino)ethyl]-2,2-diphenylacetamid, Rf: 0,61.

Beispiel 12

Analog Beispiel 10 erhält man durch Hydrierung von N-Methyl-N-((1S)-1-phenyl-2-((3S)-3-hydroxy-pyrrolidino)-ethyl]-2,2-di-phenyl-2-benzyloxy-acetamid:
N-Methyl-N-[(1S)-1-phenyl-2-((3S)3-hydroxy-pyrrolidino)-ethyl]-2,2-di-phenyl-2-hydroxy-acetamid.

Analog erhält man durch Hydrierung der entsprechenden 2,2-Diphenyl-2-benzyloxy-acetamide:

N-Methyl-N-[(1S)-1-(p-methoxy-phenyl)-2-((3S)-3-hydroxy-pyrrolidino)-ethyl]-2,2-diphenyl-2-hydroxy-acetamid;
N-Methyl-N-[(1S)-1-phenyl-2-pyrrolidino-ethyl]-2,2-diphenyl-2-hydroxy-acetamid;
N-Methyl-N-[(1S)-1-(p-methoxy-phenyl)-2-((3S)-3-hydroxy-pyrrolidino)-ethyl]-2,2-diphenyl-2-hydroxy-acetamid;
N-Methyl-N-(2-pyrrolidino-4-methyl-pentyl)-2,2-diphenyl-2-hydroxy-acetamid;
N-Methyl-N-[2-((3S)-hydroxy-pyrrolidino)-4-methyl-pentyl]-2,2-diphenyl--2-hydroxy-acetamid;
N-Methyl-N-(2-(pyrrolidino-3-methyl-butyl)-2,2-diphenyl-2-hydroxy-acetamid;
N-Methyl-N-[2-((3S)-hydroxy-pyrrolidino)-3-methyl-butyl]-2,2-diphenyl-2-hydroxy-acetamid;
N-Methyl-N-[(1S)-1-(p-hydroxy-phenyl)-2-((3S)-3-hydroxy-pyrrolidino)-ethyl]-2,2-diphenyl-2-hydroxy-acetamid;
N-Methyl-N-[(1S)-1-(p-chlor-phenyl)-2-pyrrolidino-ethyl]-2,2-diphenyl-2-hydroxy-acetamid;
N-Methyl-N-[(1S)-1-(p-methoxy-phenyl)-2-((3S)-3-hydroxy-pyrrolidino)-ethyl]-2,2-diphenyl-2-hydroxy-acetamid;
N-Methyl-N-[(1S)-1-(2,4-di-methoxy-phenyl)-2-((3S)-3-hydroxy-pyrrolidino)-ethyl]-2,2-diphenyl-2-hydroxy-acetamid;
N-Methyl-N-[(1S)-1-(2,4-di-methoxy-phenyl)-2-pyrrolidino-ethyl]-2,2-diphenyl-2-hydroxy-acetamid.

Die nachstehenden Beispiele betreffen pharmazeutische Zubereitungen.

Beispiel A: Injektionsgläser

Eine Lösung von 100 g N-Methyl-N-[1-phenyl-2(3-hydroxy-pyrrolidino)-ethyl]-diphenylessigsäureamid und 5 g Dinatrium-hydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

Beispiel B: Suppositorien

Man schmilzt ein Gemisch von 20 g 2-(2,2-Diphenyl-propionyl)-1-(1-pyrrolidinylmethyl)-1,2,3,4-tetrahydroisochinolin mit 100 g Sojalecithin und 1400 g Kakaobutter, gießt in Formen und läßt erkalten. Jedes Suppositorium enthält 20 mg Wirkstoff.

Beispiel C: Lösung

Man bereitet eine Lösung aus 1 g N-Methyl-N-[(1S)-(4-methoxy-phenyl)-2-((3S)-3-hydroxypyrrolidino)-ethyl]-2,2-diphenylacetamid, 9,38 g $NaH_2PO_4 \cdot 2\ H_2O$, 28,48 g $Na_2HPO_4 \cdot 12\ H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

Beispiel D: Salbe

Man mischt 500 mg N-Methyl-N-[(1S)-1-phenyl-2-((3S)-3-hydroxypyrrolidino)-ethyl]-2,2-diphenylacetamid mit 99,5 g Vaselin unter aseptischen Bedingungen.

Beispiel E: Tabletten

Ein Gemisch von 1 kg N-Methyl-N-[(1S)-1-phenyl-2-((3S)-hydroxy-pyrrolidino)-ethyl]-9-fluoren-carboxamid, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

**Patentansprüche**

1. Arylacetamide der Formel I

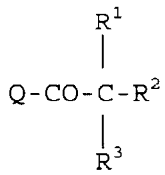

I

worin

Q $R^4$-CH($CH_2$Z)-NA-,

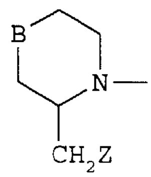

,

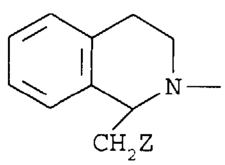

oder

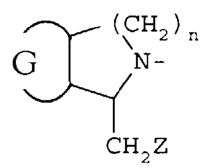

,

$R^1$ Ar,

$R^2$ Ar,

$R^1$ und $R^2$ zusammen auch

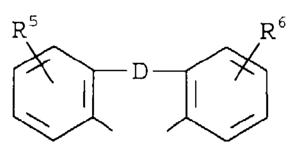

,

$R^3$ H , OH , OA oder A,

$R^4$ A oder Phenyl, welches gegebenenfalls ein- oder zweifach durch F, Cl, Br, I, OH, OA, $CF_3$, $NO_2$, $NH_2$, NHA, NHCOA, $NHSO_2A$ oder $NA_2$ substituiert sein kann,

$R^5$ und $R^6$ jeweils unabhängig voneinander H, F, Cl, Br, I, OH, OA, $CF_3$, $NH_2$, NHA, $NA_2$, NHCOA, $NHCONH_2$, $NO_2$ oder Methylendioxy,

A Alkyl mit 1-7 C-Atomen,

B $CH_2$, O, NH, NA, N-COA, N-COOA oder eine Bindung,

G ein ankondensiertes Ringsystem mit 3-5 C-Atomen, wobei gegebenenfalls ein C-Atom durch S, N oder O ersetzt sein kann und welches gegebenenfalls ein- oder zweifach durch F, Cl, Br, I, OH, OA, $NH_2$, NHA, $NA_2$, NH-COA, NA-COA oder NH-$CONH_2$ substituiert sein kann,

D $CH_2$, O, S, NH, NA, -$CH_2$-$CH_2$-, -CH=CH-, -$CH_2$O-, -$CH_2$NH-, -$CH_2$NA- oder eine Bindung,

Z 1-Pyrrolidinyl, welches gegebenenfalls einfach durch OH, OA, O-$COCH_3$ oder $CH_2OH$ substituiert sein kann,

Ar unsubstituiertes oder einfach durch $NH_2$, OH, $NHCOCH_3$, F, Cl oder $CF_3$, substituiertes Phenyl,

-alk einen Alkylenrest mit 1-7 Atomen

und

n 1 oder 2

bedeuten,
sowie deren Salze.

**2.** a) N-Methyl-N-[1-phenyl-2- (3-hydroxy-pyrrolidino)-ethyl]-2,2-diphenylacetamid;
b) 2-(2,2-Diphenylpropionyl)-1-(1-pyrrolidinylmethyl)-1,2,3,4-tetrahydroisochinolin;
c) N-Methyl-N-[(1S)-1-(4-methoxyphenyl)-2-((3S)-3-hydroxy-pyrrolidino)-ethyl]-2,2-diphenylacetamid;
d) N-Methyl-N-[(1S)-1-phenyl-2-((3S)-3-hydroxypyrrolidino)-ethyl]-2,2-diphenylacetamid;
e) N-Methyl-N- [(15) -1-phenyl-2- ((3S)-hydroxypyrrolidino)-ethyl]-9-fluoren-carboxamid;
f) N-Methyl-N-[(1S)-1-phenyl-2-pyrrolidino]-ethyl-2,2-di-(4-fluorphenyl)-acetamid;
g) N-Methyl-N-[1-(2-methylpropyl)-2-pyrrolidino-ethyl]-2,2-diphenylacetamid;
h) N-Methyl-N-[1-phenyl-2-(3-hydroxy-pyrrolidino)-ethyl]-2,2-bis-(4-fluorphenyl)-acetamid;

gemäß Anspruch 1.

**3.** Verfahren zur Herstellung eines Arylacetamids der Formel I nach Anspruch 1, dadurch gekennzeichnet, daß man eine Verbindung der Formel II

Q-H II,

worin Q die in Anspruch 1 angegebene Bedeutung hat,
mit einer Verbindung der Formel III

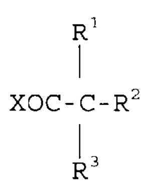

III,

worin

X Cl, Br, OH, OA, $NH_2$, $N_3$, Acyloxy, Ar-alkoxy mit 7-11 C-Atomen oder Aroyloxy mit 6-10 C-Atomen bedeutet

und
$R^1$, $R^2$ und $R^3$ die angegebenen Bedeutungen haben,
umsetzt,

oder daß man in einer Verbindung der Formel I nach Anspruch 1 einen Rest Q, $R^1$, $R^2$ und/oder $R^3$ in einen anderen Rest Q, $R^1$, $R^2$ und /oder $R^3$ umwandelt,

oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,

und/oder daß man eine basische Verbindung der Formel I durch Behandeln mit einer Säure in eines ihrer

Salze umwandelt.

4. Verfahren zur Herstellung einer pharmazeutischen Zubereitung, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

5. Pharmazeutische Zubereitung, gekennzeichnet durch einen Gehalt an mindestens einer Verbindung der Formel I, gemäß Anspruch 1, und/oder einem ihrer physiologisch unbedenklichen Salze.

6. Verwendung einer Verbindung der Formel I gemäß Anspruch 1 und/oder eines ihrer physiologisch unbedenklichen Salze zur Herstellung von Arzneimitteln.

## Claims

1. Arylacetamides of the formula I

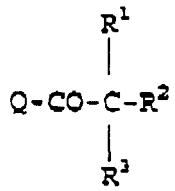

I

in which

| | |
|---|---|
| Q | is $R^4$-CH($CH_2$)-NA-, |

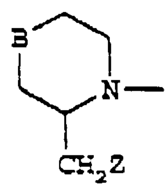

,

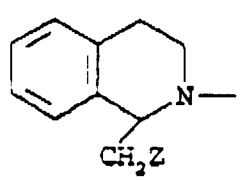

or

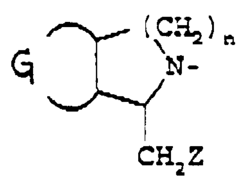

,

| | |
|---|---|
| $R^1$ | is Ar, |
| $R^2$ | is Ar, |
| $R^1$ and $R^2$ | together are also |

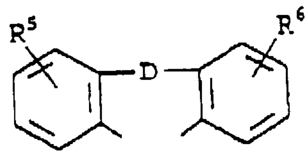

| | |
|---|---|
| $R^3$ | is H, OH, OA or A, |
| $R^4$ | is A or phenyl which can optionally be substituted once or twice by F, Cl, Br, I, OH, OA, $CF_3$, $NO_2$, $NH_2$, NHA, NHCOA, $NHSO_2A$ or $NA_2$, |
| $R^5$ and $R^6$ | are each, independently of one another, H, F, Cl, Br, I, OH, OA, $CF_3$, $NH_2$, NHA, $NA_2$, NHCOA, $NHCONH_2$, $NO_2$ or methylenedioxy, |
| A | is alkyl with 1-7 C atoms, |
| B | is $CH_2$, O, NH, NA, N-COA, N-COOA or a bond, |
| G | is a fused-on ring system with 3-5 C atoms, where one C atom can optionally be replaced by S, N or O, and which can optionally be substituted once or twice by F, Cl, Br, I, OH, OA, $NH_2$, NHA, $NA_2$, NH-COA, NA-COA or NH-$CONH_2$, |
| D | is $CH_2$, O, S, NH, NA, -$CH_2$-$CH_2$-, -CH=CH-, -$CH_2$O-, -$CH_2$NH-, -$CH_2$NA- or a bond, |
| Z | is 1-pyrrolidinyl which can optionally be substituted once by OH, OA, O-$COCH_3$ or $CH_2OH$, |
| Ar | is phenyl which is unsubstituted or monosubstituted by $NH_2$, OH, $NHCOCH_3$, F, Cl or $CF_3$, |

-alk is an alkylene radical with 1-7 C atoms and

n is 1 or 2

and the salts thereof.

**2.** a) N-methyl-N-[1-phenyl-2-(3-hydroxypyrrolidino)ethyl]-2,2-diphenylacetamide;
b) 2-(2,2-diphenylpropionyl)-1-(1-pyrrolidinylmethyl)-1,2,3,4-tetrahydroisoquinoline;
c) N-methyl-N-[(1S)-1-(4-methoxyphenyl)-2-((3S)-3-hydroxypyrrolidino)ethyl]-2,2-diphenylacetamide;
d) N-methyl-N-[(1S)-1-phenyl-2-((3S)-3-hydroxypyrrolidino)ethyl]-2,2-diphenylacetamide;
e) N-methyl-N-[(1S)-1-phenyl-2-((3S)-hydroxypyrrolidino)ethyl]-9-fluorenecarboxamide;
f) N-methyl-N-[(1S)-1-phenyl-2-pyrrolidino)-ethyl-2,2-di(4-fluorophenyl)acetamide;
g) N-methyl-N-[1-(2-methylpropyl)-2-pyrrolidinoethyl]-2,2-diphenylacetamide;
h) N-methyl-N-[1-phenyl-2-(3-hydroxypyrrolidino)ethyl]-2,2-bis(4-fluorophenyl)acetamide; according to Claim 1.

**3.** Process for the preparation of an arylacetamide of the formula I according to Claim 1, characterised in that a compound of the formula II

$$Q\text{-}H \qquad II$$

in which Q has the meaning stated in Claim 1, is reacted with a compound of the formula III

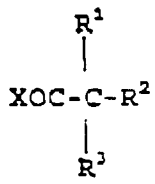

III

in which

X is Cl, Br, OH, OA, $NH_2$, $N_3$, acyloxy, Ar-alkoxy with 7-11 C atoms or aroyloxy with 6-10 C atoms,

and
$R^1$, $R^2$ and $R^3$ have the meanings stated,

or in that a radical Q, $R^1$, $R^2$ and/or $R^3$ in a compound of the formula I is converted into another radical Q, $R^1$, $R^2$ and/or $R^3$,
or in that a compound which otherwise corresponds to formula I but which, in place of one or more hydrogen atoms, contains one or more solvolysable group(s) is treated with a solvolysing agent,
and/or in that a basic compound of the formula I is converted by treatment with an acid into one of its salts.

**4.** Process for the production of a pharmaceutical preparation, characterised in that a compound of the formula I according to Claim 1 and/or one of its physiologically acceptable salts is converted together with at least one solid, liquid or semiliquid vehicle or ancillary substance into a suitable dosage form.

**5.** Pharmaceutical preparation characterised by a content of at least one compound of the formula I according to Claim 1, and/or one of its physiologically acceptable salts.

**6.** Use of a compound of the formula I according to Claim 1 and/or one of its physiologically acceptable salts for producing medicaments.

**Revendications**

**1.** Arylacétamides de formule I

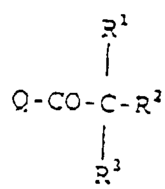

dans laquelle

Q est

$$R^4\text{-CH(CH}_2\text{Z)-NA-},$$

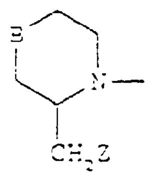

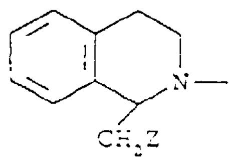 ou 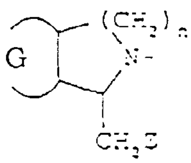

$R^1$ est Ar,
$R^2$ est Ar,
$R^1$ et $R^2$ forment ensemble aussi

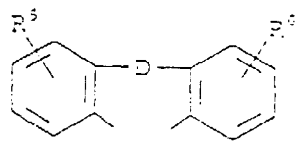

$R^3$ est H, OH, OA ou A,
$R^4$ est A ou un radical phényle, pouvant être éventuellement une ou deux fois substitué par F, Cl, Br, I, OH, OA, $CF_3$, $NO_2$, $NH_2$, NHA, NHCOA, $NHSO_2A$ ou $NA_2$,
$R^5$ et $R^6$ représentent chacun indépendamment de l'autre H, F, Cl, Br, I, OH, OA, $CF_3$, $NH_2$, NHA, $NA_2$, NHCOA, $NHCONH_2$, $NO_2$ ou le radical méthylènedioxy,
A est un groupe alkyle ayant de 1 à 7 atomes de carbone,
B est $CH_2$, O, NH, NA, N-COA, N-COOA ou une liaison,
G est un système cyclique condensé ayant de 3 à 5 atomes de carbone, dans lequel éventuellement un atome de carbone peut être remplacé par S,
N ou O, et qui peut être éventuellement substitué une ou deux fois par F, Cl, Br, I, OH, OA, $NH_2$, NHA, $NA_2$, NH-COA, NA-COA ou NH-$CONH_2$,
D est $CH_2$, O, S, NH, NA, -$CH_2$-$CH_2$-, -CH=CH-, -$CH_2$O-, -$CH_2$NH-, -$CH_2$NA- ou une liaison,
Z est un groupe 1-pyrrolidinyle, qui peut être éventuellement substitué une fois par OH, OA, O-$COCH_3$ ou $CH_2OH$,
Ar est un groupe phényle non-substitué, ou une fois substitué par $NH_2$, OH, $NHCOCH_3$, F, Cl ou $CF_3$,
-alk représente un résidu alkylène ayant de 1 à 7 atomes de carbone,

et
n vaut 1 ou 2
ainsi que ses sels.

**2.** a) N-méthyl-N-[1-phényl-2-(3-hydroxy-pyrrolidino)-éthyl]-2,2-diphénylacétamide,
b) 2-(2,2-diphénylpropionyl)-1-(1-pyrrolidinyl-méthyl)-1,2,3,4-tétrahydroisoquinoléine,
c) N-méthyl-N-[(1S)-1-(4-méthoxyphényl)-2-((3S)-3-hydroxypyrrolidino)-éthyl]-2,2-diphénylacétamide,
d) N-méthyl-N-[(1S)-1-phényl-2-((3S)-3-hydroxy-pyrrolidino)-éthyl]-2,2-diphénylacétamide,
e) N-méthyl-N-[(1S)-1-phényl-2-((3S)-3-hydroxy-pyrrolidino)-éthyl]-9-fluorène-carboxamide,
f) N-méthyl-N-[(1S)-1-phényl-2-pyrrolidino]-éthyl-2,2-di-(4-fluorophényl)-acétamide,
g) N-méthyl-N-[1-(2-méthylpropyl)-2-pyrrolidino-éthyl]-2,2-diphénylacétamide,
h) N-méthyl-N-[1-phényl-2-(3-hydroxy-pyrrolidino)-éthyl]-2,2-bis(4-fluorophényl)-acétamide,

selon la revendication 1.

**3.** Procédé pour préparer un arylacétamide de formule I selon la revendication 1, caractérisé en ce qu'on fait réagir un composé de formule II

Q-H II,

dans laquelle Q a les significations données dans la revendication 1, avec un composé de formule III

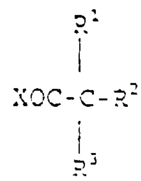

dans laquelle

X est Cl, Br, OH, OA, $NH_2$, $N_3$ ou un groupe acyloxy, Ar-alcoxy ayant de 7 à 11 atomes de carbone ou aroyloxy ayant de 6 à 10 atomes de carbone, et
$R^1$, $R^2$ et $R^3$ ont les significations indiquées,
ou en ce qu'on convertit, dans un composé de formule I selon la revendication 1, un radical Q, $R^1$, $R^2$ et/ou $R^3$ en un autre radical Q, $R^1$, $R^2$ et/ou $R^3$,
ou sinon en ce qu'on traite, avec un agent de solvolyse, un composé correspondant à la formule I mais qui, au lieu d'un ou plusieurs atomes d'hydrogène, contient un ou plusieurs groupes solvolysables,
et/ou en ce que l'on convertit un composé basique de formule I, par traitement avec un acide, en l'un de ses sels.

**4.** Procédé pour fabriquer une préparation pharmaceutique, caractérisé en ce qu'on transforme en une forme administrable appropriée un composé de formule I selon la revendication 1 et/ou l'un de ses sels inoffensifs du point de vue physiologique, avec au moins un excipient ou adjuvant solide, liquide ou semi-liquide.

**5.** Préparation pharmaceutique, caractérisée en ce qu'elle contient au moins un composé de formule I selon la revendication 1 et/ou l'un de ses sels inoffensifs du point de vue physiologique.

**6.** Utilisation d'un composé de formule I selon la revendication 1 et/ou d'un de ses sels inoffensifs du point de vue physiologique pour fabriquer des médicaments.